# EUROPEAN PATENT APPLICATION

(11) **EP 3 354 244 A1**
(43) Date of publication of application: **01.08.2018**
(21) Application number: 18151832.5
(22) Date of filing: 16.01.2018
(51) Int. Cl.: A61F 13/20, A61F 13/34, A61F 13/26

(54) **DEFORMABLE TAMPON**

(30) Priority: 25.01.2017 TW 106102776
(71) Applicant: Chien, Yuan-Cheng, Kaohsiung City (TW)
(72) Inventor: Chien, Yuan-Cheng, Kaohsiung City (TW)
(74) Representative: Viering, Jentschura & Partner mbB Patent- und Rechtsanwälte

(57) **Abstract**

A deformable tampon (4, 4', 4", 4a, 4b) has an absorbent body (41, 41', 41", 41a, 41b) and a pull string (42). The absorbent body (41, 41', 41", 41a, 41b) is configured to be inserted into a vagina (5) for absorbing menstrual blood, is made into a helical shape from at least one cotton body (411), and includes a plurality of cotton loops (412, 412', 412", 412b) extending helically around an axis (A) and in contact with each other. The pull string (42) is provided on an end of the absorbent body (41, 41', 41", 41a, 41b) for pulling the absorbent body (41, 41', 41", 41a, 41b) out of the vagina (5).

## Description

This application claims the benefit of Taiwan patent application No. 106102776, filed on January 25, 2017, the entire contents of which are incorporated herein by reference.

### 1. Field of the Invention

The present invention relates to a hygiene product, and more particularly to a deformable tampon.

### 2. Description of Related Art

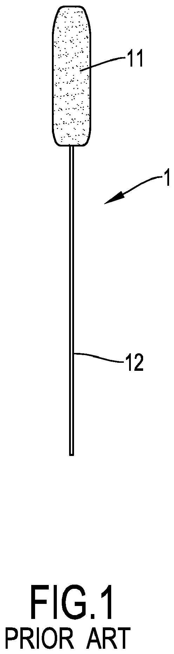

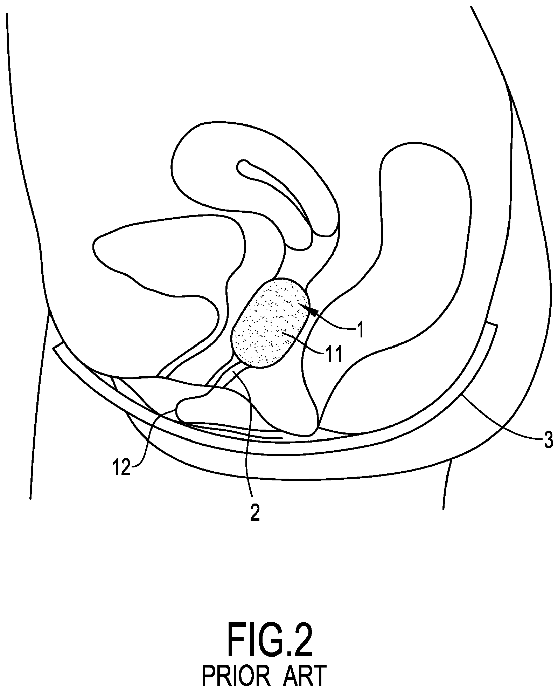

In addition to sanitary napkins, tampons are also commonly used by women. Unlike the sanitary napkins, the tampon is comfortable to use without muggy feeling and will not cause discomfort to the user during walking due to friction. With reference to Figs. 1 to 3, a conventional tampon 1 includes an absorbent body 11 having a generally cylindrical shape and a curved front end surface, and a pull string 12 connected to the absorbent body 11. In use, the absorbent body 11 is inserted into the vagina 2 for absorbing menstrual blood to achieve the effect of preventing leakage of the menstrual blood and cleaning. The pull string 12 is pulled to remove the absorbent body 11 from the vagina 2.

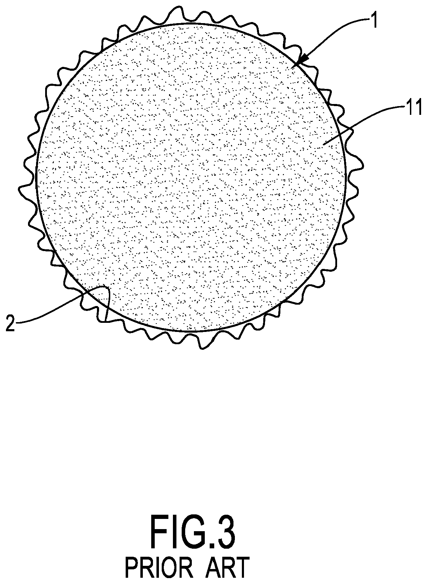

The shape of the vagina 2 is curved. After the conventional tampon 1 is inserted into the vagina 2, the absorbent body 11 will absorb the menstrual blood and expand, but it will not completely contact an inner wall of the vagina 2 which has an irregular shape, so that gaps exist between the absorbent body 11 and the inner wall of the vagina 2, as shown in Fig. 3. Because of the presence of the gaps, the menstrual blood is prone to flow out of the vagina 2 through the gaps, so that a user must also use a sanitary napkin to absorb the menstrual blood flowing out of the vagina 2. Therefore, the conventional tampon 1 is inconvenient to use, and there is still room for improvement thereof.

To overcome the shortcomings, the present invention provides a deformable tampon to mitigate or obviate the aforementioned problems.

The objective of the invention is to provide a deformable tampon that can prevent leakage of the menstrual blood.

The deformable tampon has an axis, an absorbent body, and a pull string. The absorbent body is configured to be inserted into a vagina for absorbing menstrual blood. The absorbent body is made into a helical shape from at least one cotton body, and has a plurality of cotton loops extending helically around the axis and in contact with each other. The pull string is provided on an end of said absorbent body for pulling said absorbent body out of the vagina.

The cotton loops extend helically around the axis of the deformable tampon and can bend and deform naturally to abuttingly contact the inner wall of the vagina. After absorbing the menstrual blood, the cotton loops can abut and fit with the inner wall of the vagina and increase the area of contact therewith, so that the deformable tampon can prevent leakage of the menstrual blood. The cotton loops can be easily removed from the vagina without feeling anything.

Other objectives, advantages and novel features of the invention will become more apparent from the following detailed description when taken in conjunction with the accompanying drawings.

### IN THE DRAWINGS:

Fig. 1 is a schematic view of a conventional tampon;
Fig. 2 illustrates the conventional tampon in a state of use;
Fig. 3 illustrates gaps between the conventional tampon and an inner wall of the vagina;
Fig. 4 is a perspective view of a first embodiment of a deformable tampon in accordance with the present invention;
Fig. 5 is a side view in partial section of the deformable tampon in Fig. 4;
Fig. 6 is a perspective view similar to Fig. 4, but with an auxiliary device attached to an absorbent body of the deformable tampon in Fig. 4;
Fig. 7 is a side view in partial section of the deformable tampon in Fig. 6;
Fig. 8 illustrates the first embodiment of the deformable tampon in a state of use;
Fig. 9 illustrates a state in which the absorbent body of the first embodiment of the deformable tampon absorbs menstrual blood;
Fig. 10 illustrates the absorbent body of the first embodiment of the deformable tampon before absorbing the menstrual blood;
Fig. 11 illustrates the absorbent body of the first embodiment of the deformable tampon in abutting contact with the inner wall of the vagina after absorbing the menstrual blood;
Fig. 12 is an alternative form of the first embodiment of the deformable tampon in Fig. 4;
Fig. 13 is a schematic view of a second embodiment of a deformable tampon in accordance with the present invention;
Fig. 14 is a schematic view of a third embodiment of a deformable tampon in accordance with the present invention;
Fig. 15 is a fragmentary perspective view of a fourth embodiment of a deformable tampon in accordance with the present invention;
Fig. 16 is an alternative form of the fourth embodiment of the deformable tampon in Fig. 15; and
Fig. 17 is a perspective view of a fifth embodiment of a deformable tampon in accordance with the present invention.

Before the present invention is described in greater detail with reference to the accompanying embodiment, it should be noted herein that like elements are denoted by the same reference numerals throughout the disclosure of the present invention.

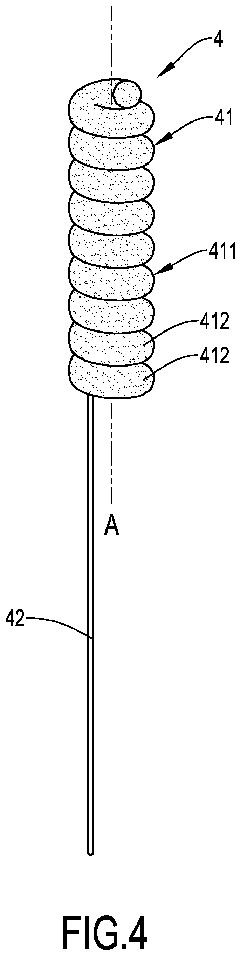

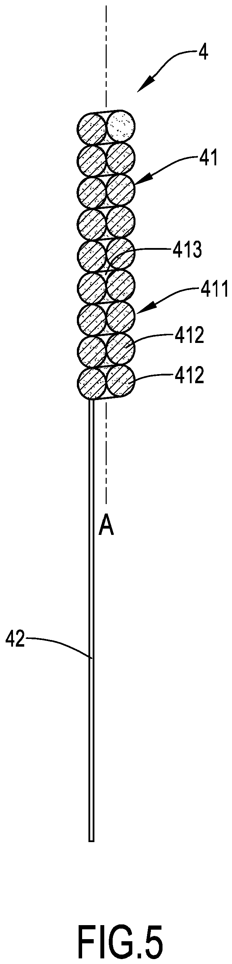

With reference to Figs. 4 and 5, a first embodiment of a deformable tampon 4 in accordance with the present invention is shown to include an absorbent body 41 and a pull string 42 provided on one end of the absorbent body 41. In the first embodiment of the present invention, the absorbent body 41 is made into a helical shape from a single cotton body 411, and includes a plurality of cotton loops 412 extending helically around an axis A. Each of two outermost ones of the cotton loops 412 has one end connected to one end of an adjacent cotton loop 412 by a meltable adhesive. As such, the cotton loops 412 can be held in abutting contact against each other to maintain the helical shape of the absorbent body 41. In the first embodiment of the deformable tampon, the cotton loops 412 have equal diameters, and cooperate with each other to define a receiving space 413 extending along the axis A.

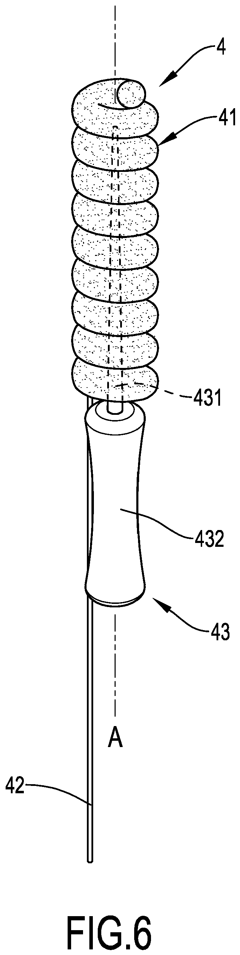

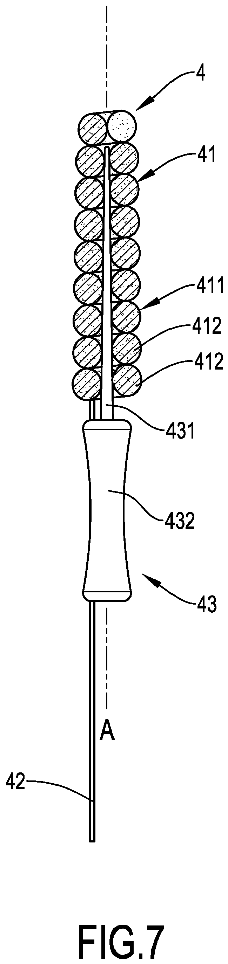

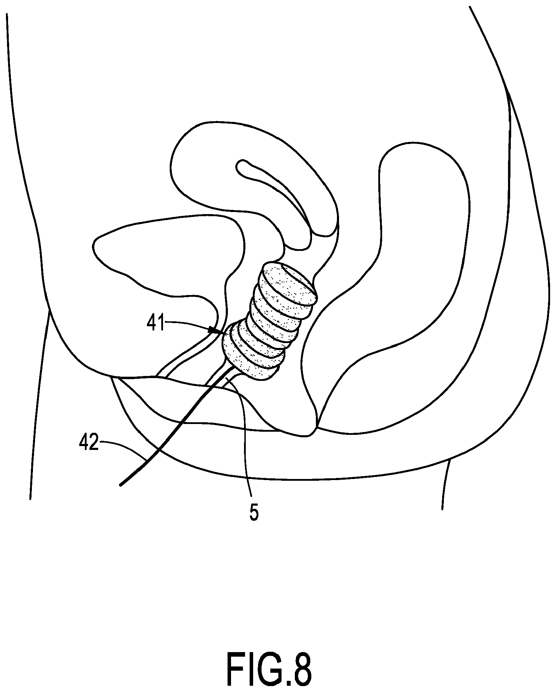

With reference to Figs. 6 and 7, in actual practice, the first embodiment of the deformable tampon further includes an auxiliary device 43. The auxiliary device 43 includes a push rod 431 removably inserted into the receiving space 413, and a handgrip 432 fixedly connected to one end of the push rod 431 and located outside of the receiving space 413. The push rod 431 has a length shorter than that of the absorbent body 41. The width of the handgrip 432 is larger than that of the push rod 431 and that of the receiving space 413, so that the handgrip 432 will not extend into the receiving space 413. The pull string 42 is tied or fixedly connected to the outermost one of the cotton loops 412 which is proximate to the handgrip 432. With reference to Fig. 8, the pull string 42 is pulled by a user to remove the absorbent body 41 from the vagina 5.

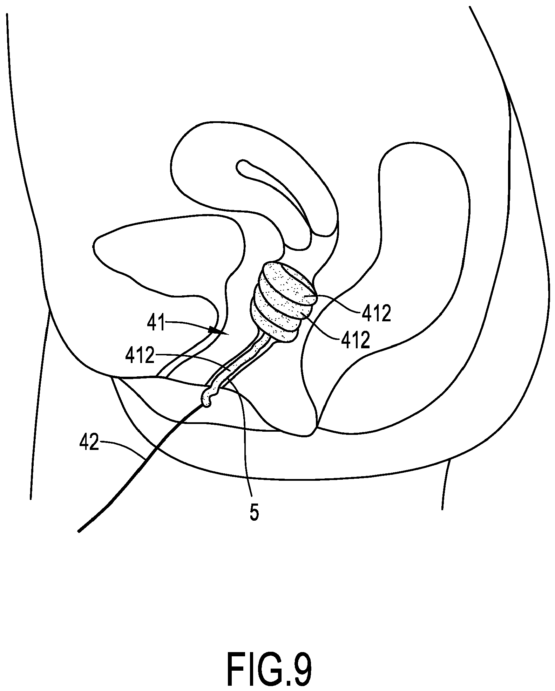

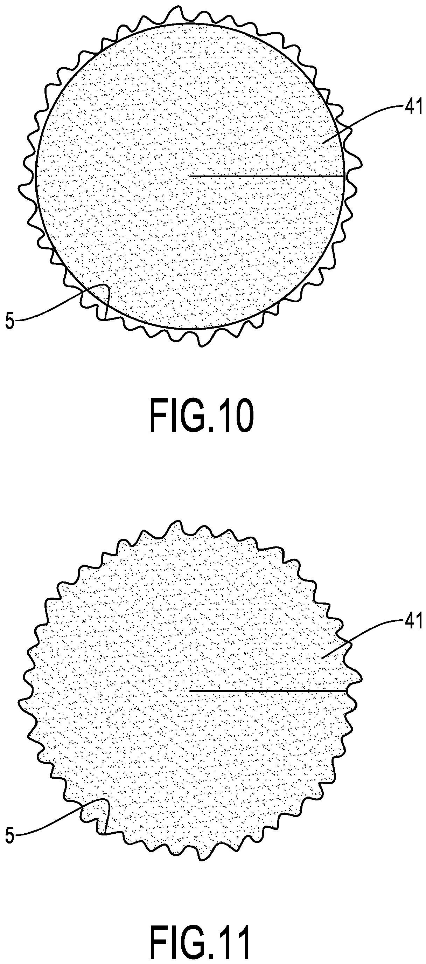

With reference to Figs. 8 and 10, in combination with Fig. 7, in use, the user grips the handgrip 432 and pushes the absorbent body 41 of the deformable tampon 4 into the vagina 5. After the absorbent body 41 is positioned in the vagina 5, the handgrip 432 is pulled away from the vagina 5 to remove the push rod 431 from the receiving space 413 of the absorbent body 41 and out of the vagina 5. The absorbent body 41 can match the shape of the vagina 5 and bends accordingly, as shown in Fig. 8. At this time, with reference to Fig. 10, gaps exist between the cotton loops 412 and an inner wall of the vagina 5. With reference to Figs. 9 and 11, in combination with Fig. 7, after absorbing the menstrual blood, and because the meltable adhesive is melted inside the vagina 5, the cotton loops 412 can bend relative to each other or move slightly deviated relative to each other along a radial direction. Such mobility can enable the cotton loops 412 to abut and fit with the uneven or curved surface of the inner wall of the vagina 5. Coupled with the expansion or swelling of the cotton loops 412 after absorbing the menstrual blood, the cotton loops 412 can deform and abuttingly contact the inner wall of the vagina 5 as shown in Fig. 11, thereby preventing existence of the gaps and leakage of the menstrual blood therefrom.

To remove the deformable tampon 4 after using it for some time, the user pulls the pull string 42 to remove the absorbent body 41 out of the vagina 5. When the absorbent body 41 is pulled out of the vagina 5, it gradually straightens out, as shown in Fig. 9, so that excessive friction with the inner wall of the vagina 5 can be prevented, thereby providing comfort to the user. It should be noted herein that when the absorbent body 41 is inserted into the vagina 5, the absorbent body 41 has a distal end opposite to a vaginal opening of the vagina 5 and a proximal end proximate to the vaginal opening of the vagina 5, and one end of the pull string 42 is fixed to the outermost one of the cotton loops 412 which is located at the proximal end of the absorbent body 41.

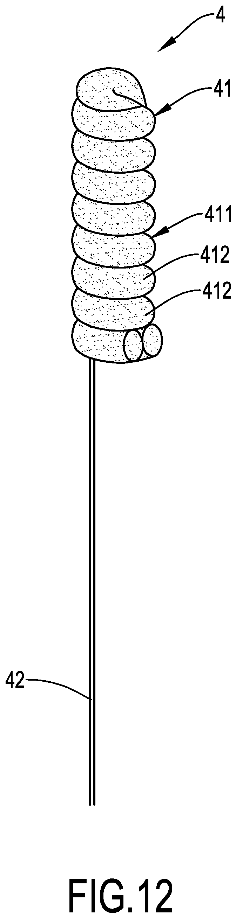

In an alternative form of the first embodiment of the deformable tampon, as shown in Fig. 12, the absorbent body 41 is bent into half, and is then made into a helical shape.

In sum, the absorbent body 41 of the deformable tampon 4 of the present invention includes the cotton loops 412 that extend helically around the axis A, that are held in abutting contact against each other, and that can bend and deform naturally to abuttingly contact the inner wall of the vagina 5 according to the shape of the vagina 5 after absorbing the menstrual blood and expanding, so that leakage of the menstrual blood can be prevented, and the cotton loops 412 can be easily removed from the vagina 5 without feeling anything. In the conventional tampon, although it expands and fills the vagina 5 after absorbing the menstrual blood, since the vaginas 5 have different sizes, different degrees of discomfort can be experienced by the user due to the expanding pressure thereof inside the vagina 5.

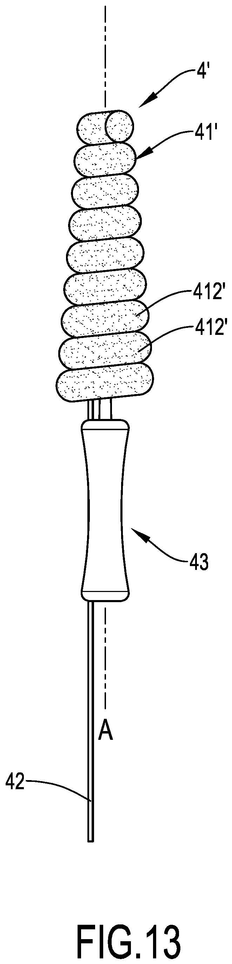

With reference to Fig. 13, the second embodiment of the deformable tampon 4' of the present invention is shown to be generally identical to the first embodiment, and differs in that, in the second embodiment, the diameters of the cotton loops 412' of the absorbent body 41' gradually decrease from one end of the absorbent body 41' which is proximate to the handgrip 432 to the other end thereof which is opposite to the handgrip 432. The pull string 42 has one end fixed to the cotton loop 412' having the largest diameter. The second embodiment provides an alternative form of the absorbent body 41' for a user to choose according to her needs.

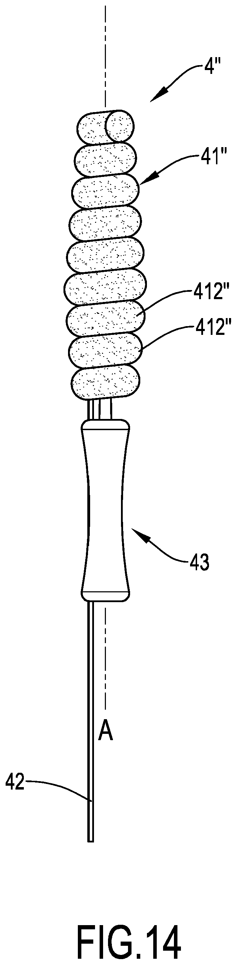

With reference to Fig. 14, the third embodiment of the deformable tampon 4" of the present invention is shown to be generally identical to the first embodiment, and differs in that, in the third embodiment, the diameters of the cotton loops 412" of the absorbent body 41" gradually increase from one end of the absorbent body 41", which is proximate to the handgrip 432, to a central portion thereof and then gradually decrease from the central portion to the other end of the absorbent body 41" which is opposite to the handgrip 432. The third embodiment provides another alternative form of the absorbent body 41" for a user to choose according to her needs.

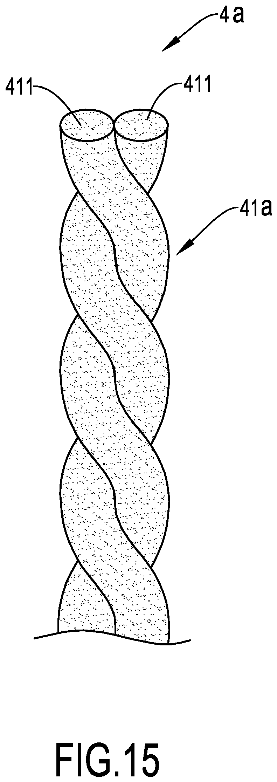

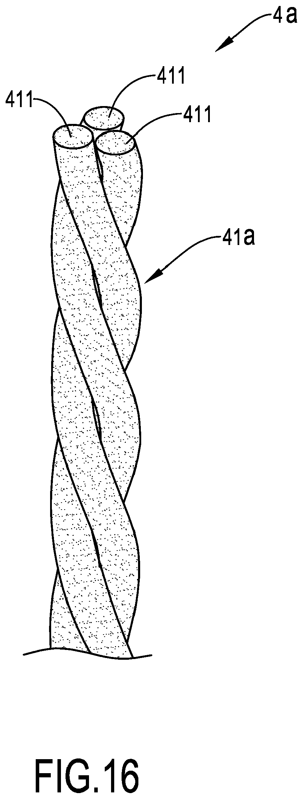

With reference to Fig. 15, the fourth embodiment of the deformable tampon 4a of the present invention is shown to be generally identical to the first embodiment, and differs in that, in the fourth embodiment, the absorbent body 41a is made into a helical shape by intertwining two cotton bodies 411. In an alternative form of the fourth embodiment, as shown in Fig. 16, the absorbent body 41a is made into a helical shape by intertwining three cotton bodies 411. However, the number of the cotton bodies 411 is not limited to what is disclosed herein. The fourth embodiment provides yet another alternative form of the absorbent body 41a for a user to choose according to her needs.

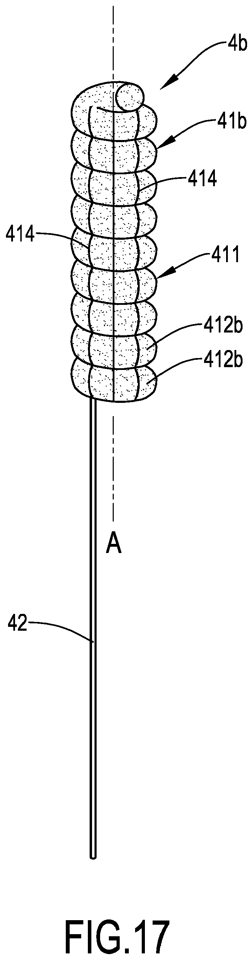

With reference to Fig. 17, the fifth embodiment of the deformable tampon 4b of the present invention is shown to be generally identical to the first embodiment, and differs in that, in the fifth embodiment, an outer circumferential surface of each cotton loop 412b is formed with a plurality of circumferentially spaced-apart grooves 414 surrounding the axis A. Each of the grooves 414 extends along a length direction of the absorbent body 41b which is parallel to the axis A. The grooves 414 of one of the cotton loops 412b are respectively aligned and communicate with the grooves 414 of an adjacent one of the cotton loops 412b. When the cotton loop 412b, which is opposite to the vagina 5, is saturated with the menstrual blood and can no longer absorb the menstrual blood, the excessive menstrual blood will flow out via the grooves 414 and be absorbed by the rest of the cotton loops 412b which are not yet saturated with the menstrual blood, thereby preventing the menstrual blood from being stuck on top of the cotton loop 412b which is opposite to the vagina 5.

In sum, through the mobility of the cotton loops 412, 412', 412", 412b after absorbing the menstrual blood, the cotton loops 412, 412', 412", 412b can abut and fit with the inner wall of the vagina 5 and increase the area of contact therewith, so that the deformable tampon 4 of the present invention can prevent leakage of the menstrual blood, thereby providing convenience and comfort during use thereof. Hence, the object of the present invention can indeed be achieved.

In the description above, for the purposes of explanation, numerous specific details have been set forth in order to provide a thorough understanding of the embodiment. It will be apparent, however, to one skilled in the art, that one or more other embodiments may be practiced without some of these specific details. It should also be appreciated that reference throughout this specification to "one embodiment," "an embodiment," an embodiment with an indication of an ordinal number and so forth means that a particular feature, structure, or characteristic may be included in the practice of the disclosure. It should be further appreciated that in the description, various features are sometimes grouped together in a single embodiment, figure, or description thereof for the purpose of streamlining the disclosure and aiding in the understanding of various inventive aspects.

While the disclosure has been described in connection with what are considered the exemplary embodiments, it is understood that this disclosure is not limited to the disclosed embodiments but is intended to cover various arrangements included within the spirit and scope of the broadest interpretation so as to encompass all such modifications and equivalent arrangements.

## Claims

1. A deformable tampon, **characterized in that** the deformable tampon comprises:
an axis (A) defined in the deformable tampon;
an absorbent body (41, 41', 41", 41a, 41b) configured to be inserted into a vagina (5) for absorbing menstrual blood, said absorbent body (41, 41', 41", 41a, 41b) being made into a helical shape from at least one cotton body (411), and including a plurality of cotton loops (412, 412', 412", 412b) extending helically around the axis (A) and in contact with each other; and
a pull string (42) provided on an end of said absorbent body (41, 41', 41", 41a, 41b) for pulling said absorbent body (41, 41', 41", 41a, 41b) out of the vagina (5).

2. The deformable tampon as claimed in claim 1, wherein said cotton loops (412, 412', 412", 412b) cooperatively define a receiving space (413) extending along the axis(A), said deformable tampon (4, 4', 4", 4a, 4b) further **characterized by** an auxiliary device (43) which includes a push rod (431) removably inserted into said receiving space (413), and a handgrip (432) connected to said push rod (431) and located outside of said receiving space (413), said handgrip (432) being operable to push said absorbent body (41, 41', 41", 41a, 41b) into the vagina (5) and to pull said push rod (431) away from said absorbent body (41, 41', 41", 41a, 41b) and out of the vagina (5).

3. The deformable tampon as claimed in claim 1, wherein said cotton loops (412, 412b) have equal diameters.

4. The deformable tampon as claimed in claim 2, wherein said cotton loops (412') have diameters gradually decreasing from one end of said absorbent body (41') which is proximate to said handgrip (432) to the other end of said absorbent body (41') which is opposite to said handgrip (432), and said pull string (42) is fixed to said cotton loop (412') having the largest diameter.

5. The deformable tampon as claimed in claim 1, wherein said cotton loops (412") have diameters gradually increasing from one end of said absorbent body (41") to a central portion thereof and then gradually decreasing from said central portion of said absorbent body (41") to the other opposite end of said absorbent body (41").

6. The deformable tampon as claimed in claim 1, wherein said absorbent body (41a) is made into a helical shape by intertwining a plurality of cotton bodies (411).

7. The deformable tampon as claimed in claim 1, wherein an outer circumferential surface of each of said cotton loops (412b) is formed with a plurality of circumferentially spaced-apart grooves (414) surrounding the axis (A), each of said grooves (414) extending along a length direction of said absorbent body (41b) which is parallel to the axis (A), said grooves (414) of one of said cotton loops (412b) being respectively aligned and communicating with said grooves (414) of an adjacent one of said cotton loops (412b).

8. The deformable tampon as claimed in claim 1, wherein said absorbent body (41, 41', 41", 41a, 41b) is inserted into the vagina (5), said absorbent body (41, 41', 41", 41a, 41b) has a distal end opposite to a vaginal opening of the vagina (5) and a proximal end proximate to the vaginal opening of the vagina (5), and one end of said pull string (42) is fixed to said cotton loop (412, 412', 412", 412b) which is located at the proximal end of said absorbent body (41, 41', 41", 41a, 41b).
